(19) Europäisches Patentamt / European Patent Office / Office européen des brevets

(11) **EP 2 906 131 B1**

(12) **EUROPÄISCHE PATENTSCHRIFT**

(45) Veröffentlichungstag und Bekanntmachung des Hinweises auf die Patenterteilung:
**12.04.2017 Patentblatt 2017/15**

(51) Int Cl.:
**A61B 17/80** *(2006.01)*　　　**A61B 17/70** *(2006.01)*
**A61B 17/86** *(2006.01)*

(21) Anmeldenummer: **13766275.5**

(22) Anmeldetag: **20.09.2013**

(86) Internationale Anmeldenummer:
**PCT/EP2013/069584**

(87) Internationale Veröffentlichungsnummer:
**WO 2014/056704 (17.04.2014 Gazette 2014/16)**

(54) **ÜBERDREHGESICHERTE KNOCHENSCHRAUBEN-FIXATIONSANORDNUNG**

BONE SCREW FIXATION ASSEMBLY PROTECTED AGAINST EXCESS TIGHTENING

AGENCEMENT DE FIXATION DE VIS À OS SÉCURISÉES POUR NE PAS SE FAUSSER EN TOURNANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.10.2012 EP 12007011**

(43) Veröffentlichungstag der Anmeldung:
**19.08.2015 Patentblatt 2015/34**

(73) Patentinhaber: FACET-LINK Inc.
**Rockaway NJ 07866 (US)**

(72) Erfinder: **JENSEN, Harm-Iven**
**24214 Noer (DE)**

(74) Vertreter: **Glawe, Delfs, Moll**
**Partnerschaft mbB**
**von Patent- und Rechtsanwälten**
**Postfach 13 03 91**
**20103 Hamburg (DE)**

(56) Entgegenhaltungen:
**DE-A1-102010 042 930　　US-A1- 2008 306 550**
**US-A1- 2009 062 862　　US-A1- 2010 324 604**

## Beschreibung

Die Erfindung betrifft eine Fixationsanordnung umfassend einen Halter mit Knochenschraube zur Befestigung. Die Fixationsanordnung dient insbesondere zur Verbindung eines Implantats mit umgebendem Knochenmaterial.

Es ist bekannt, zur hinreichend festen Befestigung von Implantaten oder zur Immobilisierung von Gelenken Knochenschrauben zu verwenden. Je nach Anwendungsfall ist hierbei eine winkelstarre Positionierung zwischen dem Implantat und der Knochenschraube ausreichend, in anderen Fällen ist aber eine winkelvariable (polyaxiale) Anordnung erforderlich. Zur Schaffung einer solchen Winkelverstellmöglichkeit ist es bekannt, zwischen dem eigentlich zu haltenden Objekt (Knochenplatte oder Implantat) und der Knochenschraube eine kalottenförmige Spannhülse vorzusehen, die in einem entsprechenden Aufnahmesitz in dem Haltekörper kippbar gelagert ist. Zur Befestigung wird die Knochenschraube durch einen Innenraum der kippbaren Spannhülse geführt, wobei das Gewinde der Knochenschraube nicht nur in den unter dem Haltekörper liegenden Knochen eingreift, sondern mit seinem oberen kopfnahen Bereich in ein entsprechendes Gegengewinde an der Innenwandung der Spannhülse greift. Beim Befestigen der Schraube folgt damit nicht nur eine Befestigung an dem Knochen, sondern die Knochenschraube verzwängt auf die Spannhülse gegenüber dem Haltekörper, so dass diese in ihrer Winkellage fixiert wird. Um die zur Verzwängung notwendige Aufspreizwirkung zu erzielen, ist der Schraubenkopf mit Übermaß relativ zur Weite des Innenraums ausgeführt. Eine derartige Befestigungsanordnung ist beschrieben in US 2005/154392 A1.

Eine Schwierigkeit bei der Handhabung einer derartigen Befestigungsanordnung besteht darin, dass dem Chirurgen nur schwerlich ein Gefühl dafür vermittelt wird, wann die Knochenschraube ausreichend fest angezogen ist. Gerade für Operationen an schwer zugänglichen Stellen oder an kleinen und damit empfindlichen Knochenteilen ist es verhältnismäßig unsicher, sich allein auf ein Gefühl für das Anzugsmoment zu verlassen. Die Verwendung eines Drehmomentschlüssels für sich schafft auch keine ausreichende Abhilfe, da das Anzugsmoment je nach Zustand des Gewindes (trocken oder durch Körperflüssigkeiten benetzt) erheblich variieren kann. Zudem wird das Anzugsdrehmoment bei der vorstehend beschriebenen Befestigungsanordnung noch dadurch verfälscht, dass zusätzliche Kraft zum Aufspreizen der Spannhülse aufgebracht werden muss.

In US 2010/324604 A1 und US 2008/306550 A1 wird jeweils eine Befestigungsanordnung beschrieben, bei der das Aufspreizen der Spannhülse mittels einer Knochenschraube erfolgt, die einen kegelstumpfförmigen Kopf aufweist. Weiter wird die Spannhülse durch seitlich angeordnete Nasen in einer Führung am Haltekörper geführt, so dass eine Verdrehung der Spannhülse gegenüber dem Haltekörper in Drehrichtung der Knochenschraube verhindert wird. Auch diese Befestigungsanordnung hat den Nachteil, dass ein Chirurg das Anzugsdrehmoment nicht bestimmen kann.

Der Erfindung liegt daher die Aufgabe zugrunde, eine Fixationsanordnung gemäß dem vorstehenden Stand der Technik dahingehend zu verbessern, dass sie einfacher mit einer reproduzierbaren Festigkeit angezogen werden kann.

Die erfindungsgemäße Lösung liegt in einer Fixationsanordnung mit den Merkmalen des unabhängigen Anspruchs. Vorteilhafte Weiterbildungen sind Gegenstand der abhängigen Ansprüche.

Bei einer Fixationsanordnung umfassend eine Knochenschraube, einen Halter und eine die Knochenschraube aufnehmende Spannhülse, wobei die Knochenschraube einen Schaft mit einem Gewinde im vorderen Bereich, einem Kopf und einer Verdickung im hinteren Bereich aufweist, an der ein Sekundärgewinde angeordnet ist, und der Halter mit einer Durchgangsöffnung und einem Aufnahmesitz versehen ist, in dem die Spannhülse schwenkbeweglich gelagert ist, die Spannhülse an ihrem Außenmantel einen radial abstehenden Vorsprung und in ihrem Innenraum mindestens einen Gang eines zum Sekundärgewinde komplementären Spanngewindes aufweist, wobei im ungespannten Zustand ein Freiweg gebildet ist durch ein Spaltmaß an der Spannhülse, ist erfindungsgemäß vorgesehen, dass der Vorsprung formschlüssig in eine Aussparung im Aufnahmesitz eingreift, und die Verdickung mit einem Konuswinkel $\beta$ konusartig nach hinten aufweitend geformt ist, wobei der Freiweg geteilt durch den Tangens des halben Konuswinkels (tan $\beta/2$) dem 0,5 bis 2,5-fachen der Gewindesteigung des Sekundärgewindes entspricht.

Nachfolgend seien einige verwendete Begriffe erläutert:

Unter "formschlüssig" wird verstanden, dass der Vorsprung derart in der Aussparung geführt ist, dass die Spannhülse bidirektional, d.h. in gegenüberliegende Drehrichtungen, gegenüber einer Verdrehung zum Aufnahmesitz gesichert ist. Dies bedeutet, dass der Vorsprung in dem Aufnahmesitz abgesehen von einem zur Montage erforderlichen geringfügigen Spiel spielfrei gehaltert ist.

In Bezug auf die Knochenschraube wird unter "vorne" die Richtung zu ihrer Spitze am Ende des Schafts und unter "hinten" die gegenüberliegende Richtung zum Kopf der Knochenschraube hin verstanden.

Unter "schwenkbeweglich" wird verstanden, dass die Achse der mittels der Spannhülse fixierten Knochenschraube einen frei einstellbaren Winkel bezogen auf eine Mittelachse der Durchgangsöffnung in der Haltung einnehmen kann. Der Einstellbereich beträgt vorzugsweise mindestens 15° in jeder Richtung und in Mittelachse der Durchgangsöffnung.

Kern der Erfindung ist der Gedanke, durch eine Kombination zweier Maßnahmen eine präzise Definition des Anzugswegs bis zum Erreichen eines festen Sitzes zu schaffen. Der erste Aspekt dieser Kombination liegt darin, dass die Spannhülse mittels des Vorsprungs drehfest gegenüber dem Halter verankert ist. Weiter weist die Spannhülse im Ausgangszustand (montiert, aber noch nicht festgezogene Knochenschraube) ein Spaltmaß zu ihrem Aufnahmesitz und/oder zu der Knochenschraube auf, wobei dieses als Freiweg bezeichnete Spaltmaß durch die konusförmige Gestaltung der Verdickung der Knochenschraube beim Anziehen der Knochenschraube sukzessive verschwindet. Die Rate, mit der der Freiweg verschwindet, hängt von dem Verdrehwinkel der Knochenschraube und der Steigung des Sekundärgewindes an der Verdickung ab. Hierbei sind der durch die Verdrehung der Knochenschraube erreichte Vorschubweg und der durch die konusförmige Gestaltung der Verdickung erreichte Expansionsweg, soweit er schließlich zum Verschwinden des Spaltes führt, nicht identisch, sondern stehen in Bezug zueinander über den Konuswinkel β. Die Erfindung hat erkannt, dass indem dieser Konuswinkel nicht arbiträr, sondern auf eine bestimmte Weise gewählt wird, eine Definition des Anzugswinkels der Schraube bis zum Verschwinden des Freiwegs erreicht werden kann, wenn - und das ist der entscheidende zweite Aspekt der Erfindung - die Spannhülse selbst bidirektional drehfest im Aufnahmesitz gehaltert ist. Denn ohne die bidirektionale, drehfeste Halterung wäre kein festes Bezugssystem gegeben, so dass der Anzugswinkel nicht sinnvoll angegeben werden kann, wenn sich nämlich die Spannhülse mitdreht. Da im praktischen Einsatz es nicht auszuschließen ist, dass ein Chirurg eine Schraube auch einmal zurückdreht, beispielsweise um einen Schraubenschlüssel neu anzusetzen, ist die bidirektionale Festlegung für die praktische Bedeutung enorm wichtig. Es ist das Verdienst der Erfindung, erkannt zu haben, dass die Bestimmung des Konuswinkels in der vorgeschriebenen Weise zwingend kombiniert werden muss mit einer bidirektional drehfesten Fixierung der Spannhülse, um eine praktisch sichere Handhabung und damit Praxistauglichkeit der Fixationsanordnung zu erreichen.

Das den Freiweg bildende Spaltmaß kann auf verschiedene Weise gebildet sein. Zweckmäßigerweise handelt es sich um ein Spiel zwischen der Spannhülse und dem Aufnahmesitz, sozusagen ein äußeres Spiel der Spannhülse. Zusätzlich oder alternativ kann das Freimaß auch gebildet sein durch ein Untermaß der Verdickung der Knochenschraube in Bezug auf die Weite des Innenraums der Spannhülse, also ein inneres Spiel. Beide können auch kombiniert sein. Damit können praxistaugliche, verhältnismäßig große Anzugswinkel von vorzugsweise mindestens 270° entsprechend einer ¾-Umdrehung erreicht werden.

Weiter können im Freiweg auch Elastizitäten der Knochenschraube, des Halters und insbesondere der Spannhülse berücksichtigt werden. Ist beispielsweise das für die Knochenschraube verwendete Material so elastische, dass sich die Verdickung unter Last komprimiert, so wäre die Kompressionsstrecke zusätzlich bei dem Freiweg zu berücksichtigen. Unbedingt erforderlich ist dies aber nicht, da für viele praktische Anwendungsfälle die verwendeten Materialien als hinreichend steif angesehen werden können.

Zweckmäßigerweise sind die Verdickung und der Kopf der Schraube miteinander kombiniert. Dies ermöglicht eine konstruktiv einfache Gestaltung der Knochenschraube. Zwingend ist die Kombination jedoch nicht.

Besonders bewährt hat sich die Erfindung bei Ausführungsformen, bei denen das Sekundärgewinde und das Gewinde am Schaft der Knochenschraube steigungsverschieden sind. Das Gewinde der Knochenschraube ist in der Regel verhältnismäßig grob (Steigung im Bereich von 1,25 bis 2,5 mm), während für das Sekundärgewinde eher ein Feingewinde in Betracht kommt. Die beiden Gewinde können auf diese Weise dank der Steigungsverschiedenheit an ihre jeweiligen Anwendungszwecke, nämlich Verankerung im Knochenmaterial einerseits bzw. Zusammenwirken mit der meist aus metallischem Material gefertigten Spannhülse andererseits, optimiert werden. Vorzugsweise sind die Steigungen der Gewinde so gewählt, dass das Sekundärgewinde eine kleinere Steigung aufweist als die Steigung des Gewindes am Schaft der Knochenschraube, und zwar vorzugsweise um das 0,4 bis 0,7-fache. Durch die Steigungsverschiedenheit werden beim Anziehen der Knochenschraube die Fragmente, welche durch die Knochenschraube verbunden werden, gegeneinander gezogen. Aus der erfindungsgemäßen Begrenzung des Anzugswinkels resultiert somit eine definierte Verspannung der beiden Knochenfragmente. Der Gefahr eines Überspannens mit dem Risiko eines Ausreißens der Knochenschraube aus dem Gewinde wird damit erfolgreich entgegengewirkt.

Zweckmäßigerweise ist die Höhe des Spanngewindes im Innenraum der Spannhülse beschränkt auf vorzugsweise nicht mehr als drei Gewindegänge, weiter vorzugsweise nicht mehr als anderthalb Gewindegänge. Mit einer solchen Gestaltung ist es besonders einfach, das vordere Gewinde der Knochenschraube durch das Gewinde am Innenmantel des Innenraums der Spannhülse zu führen, und zwar auch dann, wenn es zwar eine unterschiedliche Steigung aber denselben Durchmesser wie das Spanngewinde aufweist.

Die Spannhülse ist zweckmäßigerweise so ausgeführt, dass ihr Innenraum zylindrisch ist. Dies vereinfacht nicht nur die Fertigung, sondern bietet auch den Vorteil, dass der für die Berechnung des Anzugswegs bestimmende Konuswinkel allein durch die Verdickung der Knochenschraube bestimmt ist. Er soll andererseits aber auch nicht ausgeschlossen sein, dass der Innenraum konisch gestaltet ist und im Gegenzug der Außenmantel der Verdickung zylindrisch ausgeführt ist.

Mit Vorteil sind Spannhülse und Aufnahmesitz derart geformt, dass eine Grenzfläche zwischen ihnen kugelartig ist. Besonders zweckmäßig ist, wenn ein Außenmantel der Spannhülse sphärisch geformt ist. Damit ergibt sich eine besonders günstige Gestaltung für einen großen Schwenkwinkel der Spannhülse in ihrem Aufnahmesitz.

Die Spannhülse ist vorzugsweise einfach geschlitzt. Dies bedeutet, dass ein über die gesamte Höhe der Spannhülse verlaufender, durchgehender Schlitz vorgesehen ist. Damit ergibt sich eine gute Aufweitbarkeit, aber auch bessere Komprimierbarkeit zur Vereinfachung der Montage.

Mit Vorteil ist der Vorsprung an der Spannhülse im Bereich der größten Weite (Äquator) der Spannhülse angeordnet. Dies ergibt günstige Verkippeigenschaften unabhängig von der Richtung des Kippwinkels. Weiterhin ist der Vorsprung zweckmäßigerweise rotationssymmetrisch ausgeführt. Dies begünstigt die Gleichmäßigkeit der Verkippeigenschaften unabhängig von der Richtung des Kippwinkels. Ferner bietet diese Gestaltung des Vorsprungs den Vorzug, dass Taumelbewegungen der Spannhülse in ihrem Aufnahmesitz bei der Bewegung vermieden werden. Besonders zweckmäßig hierfür ist eine domförmige, insbesondere halbkugelige Form der Spitze des Vorsprungs. Der Vorsprung kann einen säulenartigen, insbesondere zylindrischen Unterbau aufweisen. Jedoch sollte dieser von der Höhe vorzugsweise gering sein (weniger als die Weite des Vorsprungs), um der Gefahr von Taumelbewegungen entgegenzuwirken.

Besonders zweckmäßig ist es, die im Vorsprung aufnehmende Aussparung im Aufnahmesitz schlitzartig auszuführen, wobei sich die schlitzartige Aussparung in Richtung der Mittelachse der Durchgangsöffnung erstreckt. Damit kann die Spannhülse auf einfache Weise durch Einschieben in ihre Position im Aufnahmesitz montiert werden. Gleichzeitig ist sie dort praktisch spielfrei formschlüssig gehaltert. Auf diese Weise werden einfache Montierbarkeit mit sicherer, bidirektional drehfester Halterung kombiniert.

Besonders bewährt hat es sich, einen zweiten Vorsprung diametral gegenüberliegend an der Spannhülse anzuordnen. Es versteht sich, dass dazu eine entsprechende zweite Aussparung am Aufnahmesitz vorzusehen ist. Dies ergibt eine besonders sichere Halterung der Spannhülse, wobei durch die beiden Vorsprünge die Kippachse der Spannhülse exakt definiert ist.

Die schlitzartige Form der Aussparung bietet weiter den Vorteil, dass der Vorsprung sich darin auf und ab bewegen, so dass eine zweite Kippbewegung um eine Achs orthogonal zu der vorgenannten Kippachse ermöglicht ist. Die Bewegungsfreiheit der Spannhülse und damit die Polyaxialität der Befestigung der Knochenschraube ist damit erhöht.

Die Erfindung wird nachfolgend unter Bezugnahme auf die beigefügten Zeichnungen näher erläutert, in denen ein vorteilhaftes Ausführungsbeispiel dargestellt ist. Es zeigen:

Fig. 1 eine Übersichtsdarstellung der Fixationsanordnung gemäß einem Ausführungsbeispiel der Erfindung im montierten Zustand;

Fig. 2 einen teilvergrößerten Ausschnitt aus Fig. 1;

Fig. 3 eine Schnittansicht einer Spannhülse;

Fig. 4 eine Schnittansicht einer Knochenschraube;

Fig. 5a, b schematische Darstellungen zum Freiweg und Konuswinkel;

Fig. 6 eine Montagesicht mit Darstellung des Anzugswinkels; und

Fig. 7 eine alternative Ausführungsform der Knochenschraube.

Eine erfindungsgemäße Fixationsanordnung umfasst eine Knochenschraube 1, einen Halter 2 sowie eine Spannhülse 3. Das in den Figuren dargestellt Ausführungsbeispiel stellt einen Doppelhalter dar, der zwei Knochenschrauben 1 sowie zwei Spannhülsen 3 umfasst.

Eine derartige Fixationsanordnung mit zwei Knochenschrauben 1 kann beispielsweise ausgeführt sein als ein außenbrückendes Implantat zur Verstärkung einer Lamina eines Wirbels (nicht dargestellt), insbesondere nach einer teilweisen Resektion der Lamina. Ein solches Implantat ist Gegenstand einer weiteren Anmeldung derselben Anmelderin mit dem internationalen Anmeldeaktenzeichen PCT/EP2012/001357 (Publ.-Nr. WO 2013/143558 A1).

Die Knochenschraube 1 ist an sich herkömmlich ausgeführt mit einem Schaft 10, in dessen vorderen Bereich ein Knochengewinde 11 vorgesehen ist. Das Knochengewinde 11 kann sich auch über den gesamten Schaft 10 erstrecken. Am hinteren Ende des Schafts 10 ist ein als Verdickung ausgeführter Schraubenkopf 13 vorgesehen. Er ist an seiner äußeren Stirnseite mit einer sternförmigen Vertiefung zur Aufnahme eines entsprechend ausgeführten Schraubenschlüssels (nicht dargestellt) versehen. Der Schraubenkopf 13 ist konusförmig gestaltet und weist an seinem Außenmantel 14 einen Konuswinkel $\beta$ auf. An dem Außenmantel 14 ist ein Sekundärgewinde 12 angeordnet. Es ist gleichsinnig zu dem Knochengewinde 11 ausgeführt, weist jedoch eine beträchtlich kleinere Steigung h auf (in dem dargestellten Ausführungsbeispiel nur die Hälfte der Steigung H des Knochengewindes 11). Das Sekundärgewinde 12 erstreckt sich etwa über drei Gewindegänge. Die Verdickung 15 kann auch gesondert von dem Schraubenkopf 13 ausgeführt sein (s.

Fig. 7).

Der Halter 2 weist an seinen beiden Enden je eine Durchgangsöffnung 20 zur Aufnahme einer der Knochenschrauben 1 auf. Sie ist in ihrem mittleren Bereich kugelkalottenförmig erweitert und bildet somit einen Aufnahmesitz 23 für die Spannhülse 3. An einer Seite des Aufnahmesitzes 23 ist eine schlitzartige Aussparung 24 ausgeformt, die sich mindestens über die halbe Tiefe der Durchgangsöffnung 20 erstreckt. Der Aufnahmesitz 23 dient zur schwenkbeweglichen Halterung der Spannhülse 3. Dazu weist die Spannhülse 3 einen sphärischen Außenmantel 30 auf, dessen Kontur im Wesentlichen komplementär ist zu derjenigen des Aufnahmesitzes 23. Die Spannhülse 3 weist eine zentrale Öffnung auf, die einen zylindrischen Innenraum 31 bildet. Durch diesen ist im montierten Zustand die Knochenschraube 1 gesteckt. Dabei kämmt die Knochenschraube 1 mit einem anderthalbfachen Gewindegang eines Spanngewindes 32, welches an der Wandung des Innenraums 31 angeordnet ist und komplementär ist zum Sekundärgewinde 12 der Knochenschraube 1.

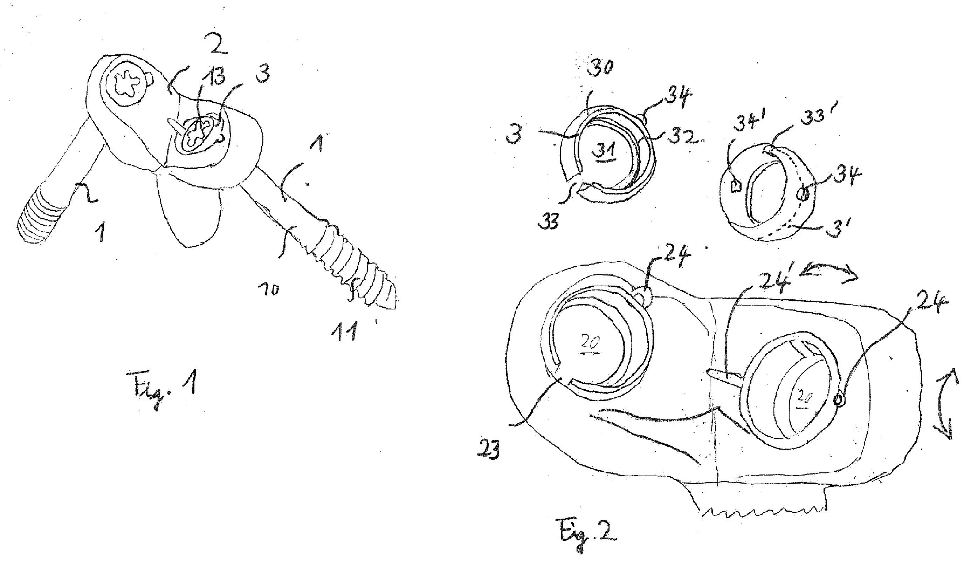

Die Spannhülse 3 weist weiter an einer Seite einen durchgehenden Schlitz 33 auf. Gegenüberliegend ist an dem Außenmantel 30 der Spannhülse 3 ein radial nach außen abragender Vorsprung 34 am Äquator angeordnet. Der Vorsprung 34 ist in dem dargestellten Ausführungsbeispiel halbkugelförmig geformt. Seine Abmessungen sind passend zu denen der schlitzartigen Aussparung 24 gewählt, so dass der Vorsprung 34 mit geringfügigem Spiel (unter geringfügig wird im Sinne dieser Anmeldung etwa ein Spiel von 5% bis 30% der Weite des Vorsprungs verstanden) formschlüssig in der schlitzartigen Aussparung 24 einschiebbar ist. Im montierten Zustand ist so die Spannhülse 3 bidirektional vor einer Verdrehung relativ zum Halter 2 gesichert. Die halbkugelförmige Gestaltung des Vorsprungs 34 erlaubt in Verbindung mit der schlitzartigen Aussparung 24 eine doppelkardanische Lagerung der Spannhülse 3 in dem Halter 2, wie sie in Fig. 2 durch die beiden Pfeile in der rechten Bildhälfte visualisiert ist. Damit ist eine achsvariable (polyaxiale) Lagerung der Knochenschraube 1 in dem Halter 2 erreicht. Praktisch wird so ein Winkelverstellbereich von ca. 15 Grad in jede Richtung ermöglicht.

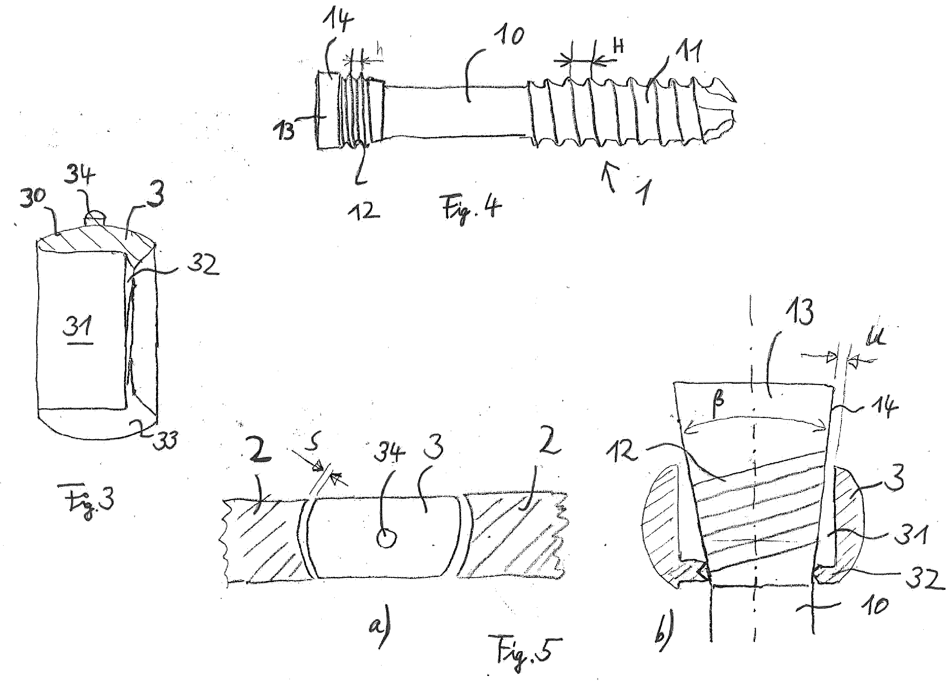

Es wird nun Bezug genommen auf die Schnittdarstellungen in Fig. 5a und 5b. Aus praktischen Gründen einer leichten Montierbarkeit und besserer Schwenkbeweglichkeit sitzt die Spannhülse 3 im Ausgangszustand mit Spiel in ihrem Aufnahmesitz 23. Das bedeutet, dass der Innendurchmesser des kugelkalottenförmigen Aufnahmesitzes 23 größer ist als der Außendurchmesser des sphärischen Außenmantels 30 der Spannhülse 3.

Weiter weist in dem dargestellten Ausführungsbeispiel die Knochenschraube 1 im Bereich der konusförmigen Verdickung, hier dem Schraubenkopf 13, ein Untermaß U auf. Das Untermaß U ist definiert als der Spalt zwischen Konusmantel 14 und dem Rand des Innenraums 31 in dem Zustand, wenn die Knochenschraube 1 mit ihrem Sekundärgewinde 12 das Spanngewinde 32 erfasst (s. Fig. 5b).

Somit ergibt sich insgesamt durch das Spiel S und das Untermass U ein Freiweg F, der durch die Summe des Spiel S und des Untermass U bestimmt ist gemäß der Beziehung

$$F = S + U.$$

Dieser Freiweg F wird beim Festziehen der Knochenschraube 1 aufgrund der konischen Aufweitung der Schraubenkopfs 13 allmählich vermindert. Ist es ganz verschwunden, dann ist die Knochenschraube 1 festgezogen. Weiteres Anziehen könnte dann zu einer Überlastung der Schraubverbindung führen, insbesondere kann es zu einem unerwünschten Ausreißen des Knochengewindes 11 aus dem Wirbelkörper kommen.

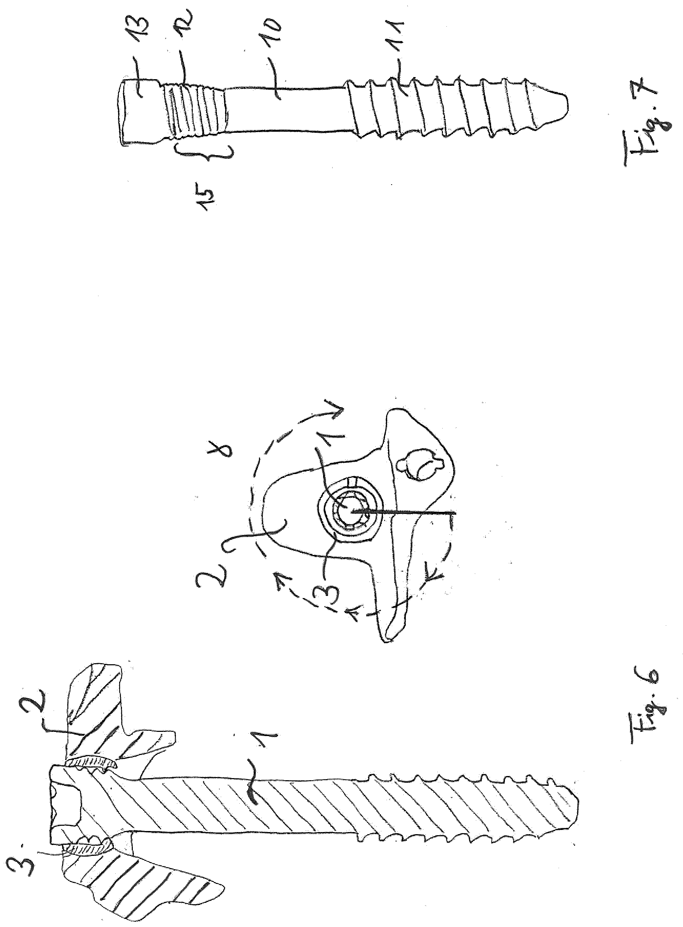

Um dies sicher zu verhindern wird ein bestimmter Anzugwinkel $\gamma$ definiert, den der Chirurg zum Festziehen anzuwenden hat. Er kann beispielsweise etwa 270 Grad betragen, also eine Dreiviertelumdrehung (s. Fig. 6). Ausgangspunkt hierfür ist die Position, bei der die Knochenschraube mit ihrem Sekundärgewinde 12 gerade in das Spanngewinde 32 eingreift (s. Fig. 5b). Ein solches Maß von 270 Grad (entsprechend einer Dreiviertelumdrehung) stellt einen für die praktische Anwendbarkeit guten Kompromiss aus guter Einstellbarkeit einerseits und verwechselungssicherer Handhabung andererseits dar. Um nun zu erreichen, dass der Freiweg F nach einer Dreiviertelumdrehung aufgezehrt ist, muss der Konuswinkel $\beta$ entsprechend gewählt sein. Erfindungsgemäß erfolgt dies gemäß der Beziehung

$$F / \tan (\beta/2) = 270°/360° * h,$$

wobei h die Steigung des Sekundärgewindes 12 ist. Zur Berechnung des gesuchten Konuswinkel $\beta$ kann die Beziehung umgestellt werden zu

$$\beta = 2 * \arctan (F / (0,75 * h)).$$

Die vorgenannte Berechnungsvorschrift für den Konuswinkel β gilt unabhängig davon, ob der Freiweg F durch das Spiel S allein oder das Untermaß U allein oder durch eine Kombination beider gebildet ist.

Die Definition des Anzugswinkels, hier im Beispiel 270 Grad für eine Dreiviertelumdrehung, ist jedoch sinnlos, wenn die Spannhülse nicht verdrehgesichert ist. Denn ohne eine Verdrehsicherung kann es zu einem Mitdrehen der Spannhülse kommen, wodurch die Definition des Anzugswinkels ihre Basis verliert. Dieser Gefahr wirkt die Erfindung entgegen, indem sie die Spannhülse 3 mittels des Vorsprungs 34 in der schlitzartigen Aussparung 24 drehfest hält, und zwar bidirektional. Erst damit wird eine praktische Umsetzbarkeit der Definition eines Anzugswinkels erreicht. Da die Spannhülse 3 drehfest an dem Halter 2 gehalten ist, bleibt der Bezugspunkt für den Anzugswinkel erhalten. Dies gilt sogar dann, wenn es zu einem an sich nicht vorgesehenen, in der Praxis aber eben doch nicht selten vorkommenden Rückdrehen kommt, zum Beispiel beim Umsetzen des Schraubenschlüssels. Erst in der Kombination mit dieser bidirektional wirkenden Halterung der Spannhülse 3 erhält das Konzept des Anzugswinkels praktische Brauchbarkeit in einem Operations-Umfeld.

Ist die Knochenschraube 1 um den bestimmten Anzugswinkel γ angezogen, wird eine doppelte Wirkung erreicht. Zum einen ist die Spannhülse 3 sicher verspannt gegen den Halter 2 und die Achse der Knochenschraube ist somit festgelegt. Zum anderen ist die Knochenschraube 1 nun fest genug angezogen für eine sichere Befestigung im Wirbel, aber auch nicht zu fest, so dass es nicht zu einer Beschädigung des Wirbels aufgrund eines wegen Überbeanspruchung ausreißenden Knochengewindes aus dem Wirbel kommen kann. Verbindet die Knochenschraube 1 zwei Knochenfragmente, so werden sie mit einer wohldefinierten Verspannung gegeneinander gezogen. Es wird also sicheres, festes Anziehen kombiniert mit einem praxistauglichen Überdrehschutz.

Bei einer alternativen Ausführungsform, wie in Fig. 2 in der rechten Bildhälfte dargestellt ist, kann vorgesehen sein, dass zwei Vorsprünge 34, 34' an einer Spannhülse 3' angeordnet sind. Dazu ist ein zweiter Vorsprung 34' vorgesehen, der identisch zu dem Vorsprung 34 ausgeführt ist. Er ist diesem diametral gegenüberliegend an dem Außenmantel der Spannhülse 3 angeordnet, weist also in die entgegengesetzte Richtung. Der Schlitz 33' ist in diesem Fall an einer Stelle zwischen den beiden Vorsprüngen 34, 34' an der Spannhülse 3 angeordnet, vorzugsweise in einer Querposition. An dem Halter 2 ist der Aufnahmesitz 23 mit einer entsprechenden zweiten schlitzartigen Aussparung 24' versehen. Mit den doppelten Vorsprüngen 34, 34' wird eine zweite Kippachse definiert, so dass eine sicher geführte doppelkardanische Beweglichkeit der Spannhülse 3 in dem Halter 2 erreicht wird.

## Patentansprüche

1. Fixationsanordnung umfassend eine Knochenschraube (1), einen Halter (2) und eine die Knochenschraube (1) aufnehmende Spannhülse (3), wobei die Knochenschraube (1) einen Schaft (10) mit einem vorderen Gewinde (11), einem Kopf (13) und einer Verdickung (15) mit einem Sekundärgewinde (12) aufweist, der Halter (2) mit einer Durchgangsöffnung (20) und einem Aufnahmesitz (23) versehen ist, in dem die Spannhülse (3) schwenkbeweglich gelagert ist, die Spannhülse (3) an ihrem Außenmantel (30) einen radial abstehenden Vorsprung (34) und in ihrem Innenraum (31) mindestens einen Gang eines zum Sekundärgewinde (12) komplementären Spanngewindes (32) aufweist, wobei im ungespannten Zustand ein Freiweg (F) durch ein Spaltmaß an der Spannhülse (3) gebildet ist, **dadurch gekennzeichnet, dass** der Vorsprung (34) formschlüssig in eine Aussparung (24) am Aufnahmesitz (23) eingreift, und die Verdickung (15) mit einem Konuswinkel (β) konusartig nach hinten aufweitend geformt ist, wobei der Freiweg (F) geteilt durch den Tangens des halben Konuswinkels (tan β/2) dem 0,5 bis 2,5-fachen der Gewindesteigung (h) des Sekundärgewindes (12) entspricht.

2. Fixationsanordnung nach Anspruch 1, **dadurch gekennzeichnet, dass** der Freiweg (F) ein Untermaß (U) umfasst, das gebildet ist durch einen Spalt zwischen Verdickung (15) und dem Innenraum (31) der Spannhülse (3).

3. Fixationsanordnung nach Anspruch 1 oder 2, **dadurch gekennzeichnet, dass** der Freiweg (F) ein Spiel (S) zwischen Spannhülse (3) und dem Aufnahmesitz (23) umfasst.

4. Fixationsanordnung nach einem der vorangehenden Ansprüche, **dadurch gekennzeichnet, dass** die Verdickung (15) und der Kopf (13) kombiniert sind.

5. Fixationsanordnung nach einem der vorangehenden Ansprüche, **dadurch gekennzeichnet, dass** das Sekundärgewinde (12) und das Gewinde (11) am Schaft (10) der Knochenschraube (1) steigungsverschieden sind.

6. Fixationsanordnung nach Anspruch 5, **dadurch gekennzeichnet, dass** das Sekundärgewinde (12) eine kleinere Steigung aufweist, vorzugsweise das 0,4 bis 0,7-fache.

7. Fixationsanordnung nach einem der vorangehenden Ansprüche, **dadurch gekennzeichnet, dass** der Innenraum (31) der Spannhülse (3) zylindrisch ist.

8. Fixationsanordnung nach einem der Ansprüche 1 bis 6, **dadurch gekennzeichnet, dass** der Innenraum der Spannhülse konisch ist und der Außenmantel der Verdickung zylindrisch ist.

9. Fixationsanordnung nach einem der vorangehenden Ansprüche, **dadurch gekennzeichnet, dass** die Höhe des Spanngewindes (32) höchstens das Dreifache, vorzugsweise das Anderthalbfache der Ganghöhe (H) des Gewindes (11) am Schaft (10) der Schraube (1) beträgt.

10. Fixationsanordnung nach einem der vorangehenden Ansprüche, **dadurch gekennzeichnet, dass** eine Grenzfläche zwischen Spannhülse (3) und Aufnahmesitz (23) kugelartig ist, insbesondere der Außenmantel (30) der Spannhülse (3) sphärisch geformt ist.

11. Fixationsanordnung nach einem der vorangehenden Ansprüche, **dadurch gekennzeichnet, dass** die Spannhülse (3) einfach geschlitzt ist.

12. Fixationsanordnung nach einem der vorangehenden Ansprüche, **dadurch gekennzeichnet, dass** der Vorsprung (34) im Bereich der größten Weite der Spannhülse (3), vorzugsweise an deren Äquator, angeordnet ist.

13. Fixationsanordnung nach einem der vorangehenden Ansprüche, **dadurch gekennzeichnet, dass** der Vorsprung (34) rotationssymmetrisch ausgeführt ist.

14. Fixationsanordnung nach Anspruch 13, **dadurch gekennzeichnet, dass** der Vorsprung (34) mit einer domförmigen, vorzugsweise halbkugeligen Spitze ausgeführt ist.

15. Fixationsanordnung nach Anspruch 13 oder 14, **dadurch gekennzeichnet, dass** der Vorsprung (34) einen säulenartigen, insbesondere zylinderförmigen Unterbau aufweist.

16. Fixationsanordnung nach einem der vorangehenden Ansprüche, **dadurch gekennzeichnet, dass** die Aussparung (24) im Aufnahmesitz (23) schlitzartig ist und sich in Richtung einer Mittelachse der Durchgangsöffnung (20) erstreckt.

17. Fixationsanordnung nach einem der vorangehenden Ansprüche, **dadurch gekennzeichnet, dass** ein zweiter Vorsprung (34') diametral gegenüberliegend angeordnet ist, wobei im Aufnahmesitz (23) eine entsprechende zweite Aussparung (24') ausgearbeitet ist.


**Claims**

1. Fixing arrangement comprising a bone screw (1), a holder (2) and a clamping sleeve (3) which receives the bone screw (1), wherein the bone screw (1) comprises a shank (10) with a front thread (11), a head (13) and a thickening (15) with a secondary thread (12), the holder (2) is provided with a through-opening (20) and a receiving seat (23) in which the clamping sleeve (3) is mounted so as to be pivotably movable, the clamping sleeve (3) comprises on its outside surface (30) a radially protruding projection (34) and in its interior (31) at least one thread of a clamping thread (32) which is complementary to the secondary thread (12), wherein in the non-clamped state a free play (F) is formed by a clearance on the clamping sleeve (3),
**characterized in that**
the projection (34) engages in a positive locking manner into a recess (24) on the receiving seat (23), and the thickening (15) is formed widening toward the rear in a cone-like manner with a cone angle ($\beta$), wherein the free play (F) divided by the tangent of half the cone angle ($\tan \beta/2$) corresponds to between 0.5 and 2.5 times the thread pitch (h) of the secondary thread (12).

2. Fixing arrangement according to Claim 1, **characterized in that** the free play (F) includes an undersize (U) which is formed by a gap between the thickening (15) and the interior (31) of the clamping sleeve (3).

3. Fixing arrangement according to Claim 1 or 2, **characterized in that** the free play (F) includes a clearance (S) between the clamping sleeve (3) and the receiving seat (23).

4. Fixing arrangement according to one of the preceding claims, **characterized in that** the thickening (15) and the head (13) are combined.

5. Fixing arrangement according to one of the preceding claims, **characterized in that** the secondary thread (12) and the thread (11) on the shank (10) of the bone screw (1) have different pitches.

6. Fixing arrangement according to Claim 5, **characterized in that** the secondary thread (12) comprises a smaller pitch, preferably between 0.4 and 0.7 times smaller.

7. Fixing arrangement according to one of the preceding claims, **characterized in that** the interior (31) of the clamping sleeve (3) is cylindrical.

8. Fixing arrangement according to one of Claims 1 to 6, **characterized in that** the interior of the clamping sleeve is conical and the outside surface of the thickening is cylindrical.

9. Fixing arrangement according to one of the preceding claims, **characterized in that** the pitch of the clamping thread (32) is a maximum of three times, preferably one and half times the pitch (H) of the thread (11) on the shank (10) of the screw (1) .

10. Fixing arrangement according to one of the preceding claims, **characterized in that** a boundary face between the clamping sleeve (3) and the receiving seat (23) is sphere-like, in particular the outside surface (30) of the clamping sleeve (3) is formed in a spherical manner.

11. Fixing arrangement according to one of the preceding claims, **characterized in that** the clamping sleeve (3) is slotted once.

12. Fixing arrangement according to one of the preceding claims, **characterized in that** the projection (34) is arranged in the region of the greatest width of the clamping sleeve (3), preferably on the equator thereof.

13. Fixing arrangement according to one of the preceding claims, **characterized in that** the projection (34) is realized so as to be rotationally symmetrical.

14. Fixing arrangement according to Claim 13, **characterized in that** the projection (34) is realized with a dome-shaped, preferably semispherical tip.

15. Fixing arrangement according to Claim 13 or 14, **characterized in that** the projection (34) comprises a column-like, in particular cylindrical substructure.

16. Fixing arrangement according to one of the preceding claims, **characterized in that** the recess (24) in the receiving seat (23) is slot-like and extends in the direction of a center axis of the through-passage (20).

17. Fixing arrangement according to one of the preceding claims, **characterized in that** a second projection (34') is arranged diametrically opposite, wherein a corresponding second recess (24') is provided in the receiving seat (23).

**Revendications**

1. Agencement de fixation comprenant une vis à os (1), un support (2) et une douille de serrage (3) contenant la vis à os (1), dans lequel la vis à os (1) présente une tige (10) avec un filet avant (11), une tête (13) et une surépaisseur (15) avec un filet secondaire (12), le support (2) est doté d'une ouverture de passage (20) et d'un siège de réception (23), dans lequel la douille de serrage (3) est montée avec un mouvement pivotant, la douille de serrage (3) présente à sa surface latérale extérieure (30) une saillie (34) saillante radialement et dans son espace intérieur (31) au moins une entrée d'un filet de serrage (32) complémentaire au filet secondaire (12), dans lequel un chemin libre (F) est formé à l'état desserré sur la douille de serrage (3) par une dimension de fente,
**caractérisé en ce que** la saillie (34) s'engage par emboîtement dans un évidement (24) sur le siège de réception (23), et la surépaisseur (15) est façonnée en cône orienté vers l'arrière avec un angle de cône (β), dans lequel le chemin libre (F) divisé par la tangente du demi-angle de cône (β/2) correspond à 0,5 à 2,5 fois le pas de filet (h) du filet secondaire (12).

**2.** Agencement de fixation selon la revendication 1, **caractérisé en ce que** le chemin libre (F) présente une sous-dimension (U), qui est formée par une fente entre la surépaisseur (15) et l'espace intérieur (31) de la douille de serrage (3).

**3.** Agencement de fixation selon la revendication 1 ou 2, **caractérisé en ce que** le chemin libre (F) comprend un jeu (S) entre la douille de serrage (3) et le siège de réception (23).

**4.** Agencement de fixation selon l'une quelconque des revendications précédentes, **caractérisé en ce que** la surépaisseur (15) et la tête (13) sont combinées.

**5.** Agencement de fixation selon l'une quelconque des revendications précédentes, **caractérisé en ce que** le filet secondaire (12) et le filet (11) sur la tige (10) de la vis à os (1) présentent un pas différent.

**6.** Agencement de fixation selon la revendication 5, **caractérisé en ce que** le filet secondaire (12) présente un pas plus petit, de préférence de 0,4 à 0,7 fois.

**7.** Agencement de fixation selon l'une quelconque des revendications précédentes, **caractérisé en ce que** l'espace intérieur (31) de la douille de serrage (3) est cylindrique.

**8.** Agencement de fixation selon l'une quelconque des revendications 1 à 6, **caractérisé en ce que** l'espace intérieur de la douille de serrage est conique, et la surface latérale de la surépaisseur est cylindrique.

**9.** Agencement de fixation selon l'une quelconque des revendications précédentes, **caractérisé en ce que** la hauteur du filet de serrage (32) vaut au maximum trois fois, de préférence une fois et demi le pas (H) du filet (11) sur la tige (10) de la vis (1).

**10.** Agencement de fixation selon l'une quelconque des revendications précédentes, **caractérisé en ce qu'**une surface limite entre la douille de serrage (3) et le siège de réception (23) est de type sphérique, en particulier la surface latérale (30) de la douille de serrage (3) est de forme sphérique.

**11.** Agencement de fixation selon l'une quelconque des revendications précédentes, **caractérisé en ce que** la douille de serrage (3) est fendue une fois.

**12.** Agencement de fixation selon l'une quelconque des revendications précédentes, **caractérisé en ce que** la saillie (34) est disposée de la région de la plus grande largeur de la douille de serrage (3), de préférence à son équateur.

**13.** Agencement de fixation selon l'une quelconque des revendications précédentes, **caractérisé en ce que** la saillie (34) est de forme symétrique de révolution.

**14.** Agencement de fixation selon la revendication 13, **caractérisé en ce que** la saillie (34) est réalisée avec une pointe en forme de dôme, de préférence en forme hémisphérique.

**15.** Agencement de fixation selon la revendication 13 ou 14, **caractérisé en ce que** la saillie (34) présente une base en forme de colonne, en particulier de forme cylindrique.

**16.** Agencement de fixation selon l'une quelconque des revendications précédentes, **caractérisé en ce que** l'évidement (24) dans le siège de réception (23) est réalisé en forme de fente et s'étend en direction d'un axe central de l'ouverture de passage (20).

**17.** Agencement de fixation selon l'une quelconque des revendications précédentes, **caractérisé en ce qu'**une deuxième saillie (34') est disposée en position diamétralement opposée, dans lequel un deuxième évidement correspondant (24') est pratiqué dans le siège de réception (23).

Fig. 2

Fig. 1

Fig. 4

Fig. 5

Fig. 3

Fig. 7

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2005154392 A1
- US 2010324604 A1
- US 2008306550 A1
- EP 2012001357 W
- WO 2013143558 A1